# EUROPEAN PATENT SPECIFICATION

(11) **EP 3 459 538 B1**
(45) Date of publication and mention of the grant of the patent: **06.05.2020**
(21) Application number: 17192115.8
(22) Date of filing: 20.09.2017
(51) Int. Cl.: A61K 31/165, C07C 237/06, A61K 47/54, A61P 3/04, A61P 25/00

(54) **CRYSTALLINE SALTS OF A DEXTROAMPHETAMINE PRODRUG**
KRISTALLINE SALZE EINER DEXTROAMPHETAMIN-PRODRUG
SELS CRISTALLINS D'UN PROMÉDICAMENT DE DEXTROAMPHÉTAMINE

(43) Date of publication of application: 27.03.2019
(73) Proprietor: Sandoz AG, 4056 Basel (CH)
(72) Inventor: LENGAUER, Hannes, 6250 Kundl (AT); PICHLER, Arthur, 6250 Kundl (AT); MARGREITER, Renate, 6250 Kundl (AT)
(74) Representative: Kluschanzoff, Harald

(56) References cited:
- WO-A1-2010/148305
- DATABASE CAPLUS [Online] CHEMICAL ABSTRACTS SERVICE, COLUMBUS, OHIO, US; 1965, "Amides from amino acids with amphetamine", XP002778249, retrieved from STN Database accession no. 1966:19805 -& BE 658 748 A 26 July 1965 (1965-07-26) -& DATABASE WPI Week 1968 1968 Thomson Scientific, London, GB; AN 1966-17758F XP002778250, & NL 6 414 901 A (HOFFMANN-LA ROCHE) 28 July 1965 (1965-07-28)

## Description

### FIELD OF THE INVENTION

The present invention relates to crystalline salts of *L*-lysine-*d*-amphetamine and to a pharmaceutical composition comprising one or more of said crystalline salts, preferably in a predetermined and/or effective amount, and at least one pharmaceutically acceptable excipient. The pharmaceutical composition of the present invention can be used as a medicament, in particular for the treatment of Attention Deficit and Hyperactivity Disorder (ADHD) and Binge Eating Disorder (BED) symptoms.

### BACKGROUND OF THE INVENTION

*L*-lysine-dextroamphetamine also known as *L*-lysine-*d*-amphetamine or lisdexamphetamine is chemically designated as (*S*)-2,6-diamino-*N*-((*S*)-1-phenylpropan-2-yl)hexanamide and can be represented by the chemical structure as depicted in formula (I)

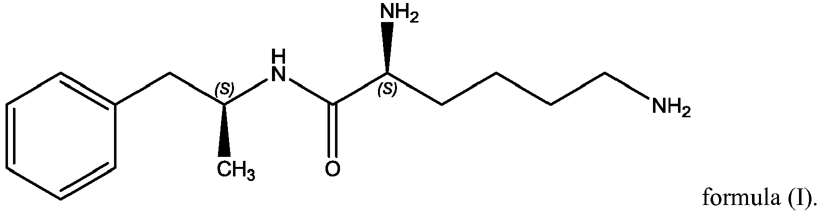

It is a long-acting prodrug of dextroamphetamine (*d*-amphetamine) used for the once-daily treatment of Attention Deficit and Hyperactivity Disorder (ADHD) and Binge Eating Disorder (BED) symptoms. ADHD is a neuropsychiatric disorder characterized by a pattern of inattention and/or hyperactivity/impulsivity. BED is a neuropsychiatric disorder characterized by episodes of compulsive eating occurring at least once a week for three months, together with a feeling of lack of control and no regular compensatory behaviours *[*E. Comiran et al. "Lisdexamfetamine: A pharmacokinetic review", European Journal of Pharmaceutical Sciences 89 (2016) 172-179*].* After oral administration and absorption of *L*-lysine-*d-*amphetamine, enzymatic hydrolysis of the peptide bond releases the active metabolite *d-*amphetamine and also yields *L*-lysine as byproduct *[*J.C. Ermer et al. "Lisdexamfetamine Dimesylate: Prodrug Delivery, Amphetamine Exposure and Duration of Efficacy" Clin Drug Investig (2016) 36:341-356*].*

*L*-lysine-*d*-amphetamine and its respective dimesylate and dihydrochloride salts are disclosed in WO 2005/000334 A1.

WO 2006/121552 A2, WO 2010/148305 A1 and WO 2017/003721 A1 concern methods for preparing amphetamine prodrugs including long lists of acids, which may be used for the preparation of pharmaceutically acceptable salts of such compounds. Concrete examples of pharmaceutical salts of *L*-lysine-*d*-amphetamine are limited to the dimesylate and the dihydrochloride salt though, whereat the dimesylate is said to be the preferred salt of *L*-lysine-*d*-amphetamine (see e.g.WO 2006/121552 A2, paragraph [098] and WO 2017/003721 A1, page 8, lines 16-18). It is noteworthy, that commercial products such as Elvanse®, Vyvanse®, Tyvense®, Elvanse Adult® and Tyvense Adult® all contain *L*-lysine-*d*-amphetamine dimesylate.

WO 2014/168727 A2 is directed at fixed dosage units of *L*-lysine-*d*-amphetamine with an atypical antipsychotic. Again, the application provides in paragraph [0048] an exemplary list of pharmaceutically acceptable salts. But concrete examples are restricted to *L*-lysine-*d-*amphetamine dimesylate only, although *L*-lysine-*d*-amphetamine sulfate and L-lysine-d-amphetamine hydrochloride are also cited in the claims.

The dioxalate salt with CAS RN 4907-02-2 for a different stereoisomer of Lisdexamfetamine is disclosed in BE 658 748 A.

Different solid-state forms of an active pharmaceutical ingredient often possess different properties. Differences in the physicochemical properties of solid-state forms can be important for the improvement of pharmaceutical compositions, for example, pharmaceutical formulations with improved dissolution profile or with improved stability or shelf-life can become accessible due to an improved solid-state form of an active pharmaceutical ingredient.

Also processing or handling of the active pharmaceutical ingredient during the formulation process may be improved. New solid-state forms of an active pharmaceutical ingredient can thus have desirable processing properties. They can be easier to handle, better suited for storage, and/or allow for better purification, compared to previously known solid-state forms.

The inventors of the present invention for example found that the dimesylate salt of *L*-lysine-*d*-amphetamine is very hygroscopic and liquefies already at moderate relative humidity levels. Said properties are critical with regard to production, pharmaceutical processing and storage and require strict control of the humidity levels of the surrounding atmosphere during all process steps and storage in order to ensure reliable efficacy and safety of the drug product containing *L*-lysine-*d*-amphetamine dimesylate.

It was thus an objective of the present invention to provide improved solid-state forms *of L-*lysine-*d*-amphetamine, in particular solid-state forms which are less hygroscopic and physically more stable against moisture.

### SUMMARY OF THE INVENTION

The invention solves one or more of the above defined objectives by providing crystalline salts of *L*-lysine-*d*-amphetamine represented by the chemical structure as depicted in formula (II) 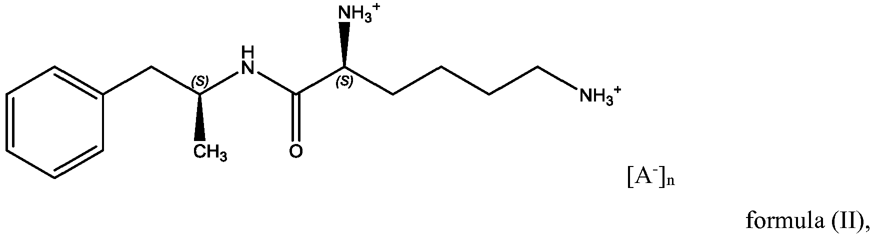 wherein n is in the range of from 1.7 to 2.3, preferably of from 1.8 to 2.2, more preferably of from 1.9 to 2.1, even more preferably of from 1.95 to 2.05 and most preferably n is 2.0 and A is selected from the group consisting of adipic acid, *p*-toluenesulfonic acid, fumaric acid and oxalic acid as defined in the claims.

The crystalline salts of the present invention are less hygroscopic and more stable against moisture compared to *L*-lysine-*d*-amphetamine dimesylate and are therefore easier to handle during production, pharmaceutical processing and storage.

### Definitions

The terms "*L*-lysine-dextroamphetamine", "*L*-lysine-*d*-amphetamine" and "lisdexamphetamine" which may be used interchangeably herein refer to (*S*)-2,6-diamino-*N-*((*S*)-1-phenylpropan-2-yl)hexanamide according to the chemical structure as depicted in formula (I) above.

The term "*L*-lysine-*d*-amphetamine dimesylate" as used herein refers to the dimesylate salt of *L*-lysine-*d*-amphetamine, having a chemical structure, wherein about one mol of *L*-lysine-*d-*amphetamine is associated with about two mol of methane sulfonic acid via ionic interactions.

The term "*L*-lysine-*d*-amphetamine dihydrochloride" as used herein refers to the dihydrochloride salt of *L*-lysine-*d*-amphetamine, having a chemical structure, wherein about one mol of *L*-lysine-*d*-amphetamine is associated with about two mol of hydrochloric acid via ionic interactions.

The term "*L*-lysine-*d*-amphetamine diadipate" as used herein refers to the diadipate salt of *L-*lysine-*d*-amphetamine, having a chemical structure as depicted in formula (I), wherein about one mol of *L*-lysine-*d*-amphetamine is associated with about two mol of adipic acid via ionic interactions. Adipic acid, also known under the chemical name hexane-1,6-dicarboxylic acid is represented by the chemical structure as depicted in formula (a)

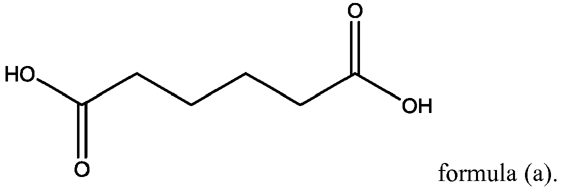

The term "*L*-lysine-*d*-amphetamine ditosylate" as used herein refers to the ditosylate salt of *L-*lysine-*d*-amphetamine, having a chemical structure as depicted in formula (IIb), wherein about one mol of *L*-lysine-*d*-amphetamine is associated with about two mol of *p*-toluenesulfonic acid via ionic interactions. *p*-Toluenesulfonic acid, also known under the chemical name 4-methylbenzene-1-sulfonic acid is represented by the chemical structure as depicted in formula (b)

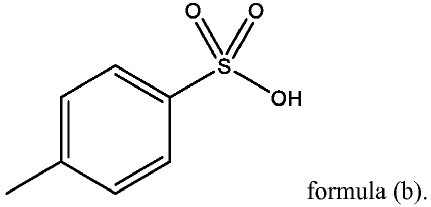

The term "*L*-lysine-*d*-amphetamine difumarate" as used herein refers to the difumarate salt of *L*-lysine-*d*-amphetamine, having a chemical structure as depicted in formula (IIc), wherein about one mol of *L*-lysine-*d*-amphetamine is associated with about two mol of fumaric acid via ionic interactions. Fumaric acid is also known under the chemical name *trans*-butenedioic acid and can be represented by the chemical structure as depicted in formula (c)

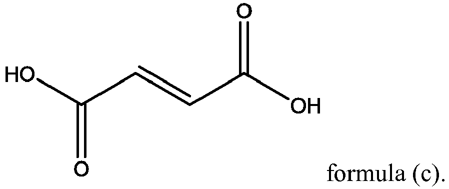

The term "*L*-lysine-*d*-amphetamine dioxalate" as used herein refers to the dioxalate salt of *L-*lysine-*d*-amphetamine, having a chemical structure as depicted in formula (IId), wherein about one mol of *L*-lysine-*d*-amphetamine is associated with about two mol of oxalic acid via ionic interactions. Oxalic acid is also known under the chemical name ethanedioic acid and can be represented by the chemical structure as depicted in formula (d)

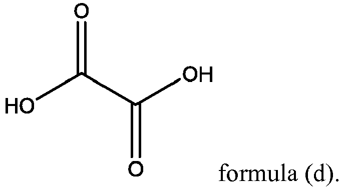

As used herein, the term "measured at a temperature in the range of from 20 to 30 °C" refers to a measurement under standard conditions. Typically, standard conditions mean a temperature in the range of from 20 to 30 °C, i.e. at room temperature. Standard conditions can mean a temperature of about 22 °C.

The term "reflection" with regard to powder X-ray diffraction as used herein, means peaks in an X-ray diffractogram, which are caused at certain diffraction angles (Bragg angles) by constructive interference from X-rays scattered by parallel planes of atoms in solid material, which are distributed in an ordered and repetitive pattern in a long-range positional order. Such a solid material is classified as crystalline material, whereas amorphous material is defined as solid material, which lacks long-range order and only displays short-range order, thus resulting in broad scattering. According to literature, long-range order e.g. extends over approximately 100 to 1000 atoms, whereas short-range order is over a few atoms only (see *"*Fundamentals of Powder Diffraction and Structural Characterization of Materials" by Vitalij K. Pecharsky and Peter Y. Zavalij, Kluwer Academic Publishers, 2003, page 3).

The term "essentially the same" with reference to powder X-ray diffraction means that variabilities in reflection positions and relative intensities of the reflections are to be taken into account. For example, a typical precision of the 2-Theta values is in the range of ± 0.2° 2-Theta. Thus, a reflection that usually appears at 4.7° 2-Theta for example can appear between 4.5° and 4.9° 2-Theta on most X-ray diffractometers under standard conditions. Furthermore, one skilled in the art will appreciate that relative reflection intensities will show inter-apparatus variability as well as variability due to degree of crystallinity, preferred orientation, sample preparation and other factors known to those skilled in the art and should be taken as qualitative measure only.

The term "solid-state form" as used herein refers to any crystalline and/or amorphous phase of a compound.

Crystalline salts of *L*-lysine-*d*-amphetamine may be referred to herein as being characterized by a powder X-ray diffractogram "as shown in" a figure. The person skilled in the art understands that factors such as variations in instrument type, response and variations in sample directionality, sample concentration, sample purity, sample history and sample preparation may lead to variations, for example relating to the exact reflection positions and intensities. However, a comparison of the graphical data in the figures herein with the graphical data generated for an unknown physical form and the confirmation that two sets of graphical data relate to the same crystal form is well within the knowledge of a person skilled in the art.

A "predetermined amount" as used herein with regard to a crystalline salt of *L-*lysine-*d-*amphetamine refers to the initial amount of said crystalline salt used for the preparation of a pharmaceutical composition having a desired dosage strength of *L*-lysine-*d*-amphetamine.

The term "effective amount" as used herein with regard to a crystalline salt of *L*-lysine-*d-*amphetamine encompasses an amount of said crystalline salt, which causes the desired therapeutic effect.

As used herein, the term "about" means within a statistically meaningful range of a value. Such a range can be within an order of magnitude, typically within 10%, more typically within 5%, even more typically within 1% and most typically within 0.1% of the indicated value or range. Sometimes, such a range can lie within the experimental error, typical of standard methods used for the measurement and/or determination of a given value or range.

The term "pharmaceutically acceptable excipient" as used herein refers to substances, which do not show a significant pharmacological activity at the given dose and that are added to a pharmaceutical composition in addition to the active pharmaceutical ingredient. Excipients may take the function of vehicle, diluent, release agent, disintegrating agent, dissolution modifying agent, absorption enhancer, stabilizer or a manufacturing aid among others. Excipients may include fillers (diluents), binders, disintegrants, lubricants and glidants.

The terms "filler" or "diluent" as used herein refer to substances that are used to dilute the active pharmaceutical ingredient prior to delivery. Diluents and fillers can also serve as stabilizers.

As used herein the term "binder" refers to substances which bind the active pharmaceutical ingredient and pharmaceutically acceptable excipient together to maintain cohesive and discrete portions.

The terms "disintegrant" or "disintegrating agent" which may be used herein interchangeably refer to substances which, upon addition to a solid pharmaceutical composition, facilitate its break-up or disintegration after administration and permit the release of the active pharmaceutical ingredient as efficiently as possible to allow for its rapid dissolution.

The term "lubricant" as used herein refers to substances which are added to a powder blend to prevent the compacted powder mass from sticking to the equipment during tableting or encapsulation process. They aid the ejection of the tablet from the dies and can improve powder flow.

The term "glidant" as used herein refers to substances which are used for tablet and capsule formulations in order to improve flow properties during capsule filling and tablet compression and to produce an anti-caking effect.

### BRIEF DESCRIPTION OF THE FIGURES

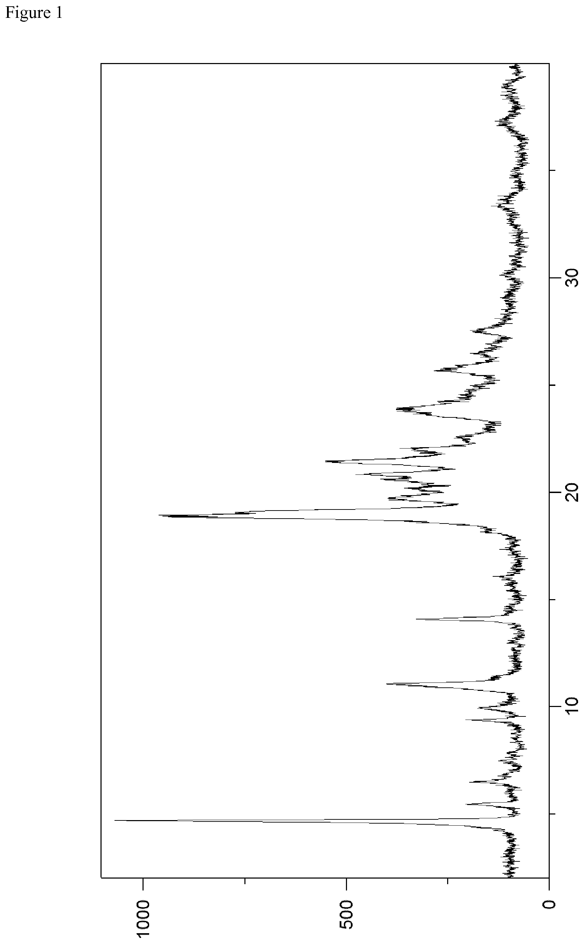

**Figure 1****:** illustrates a representative powder X-ray diffractogram of crystalline *L*-lysine-*d-*amphetamine diadipate form I of the present invention. The x-axis shows the scattering angle in °2-Theta, the y-axis shows the intensity of the scattered X-ray beam in counts of detected photons.

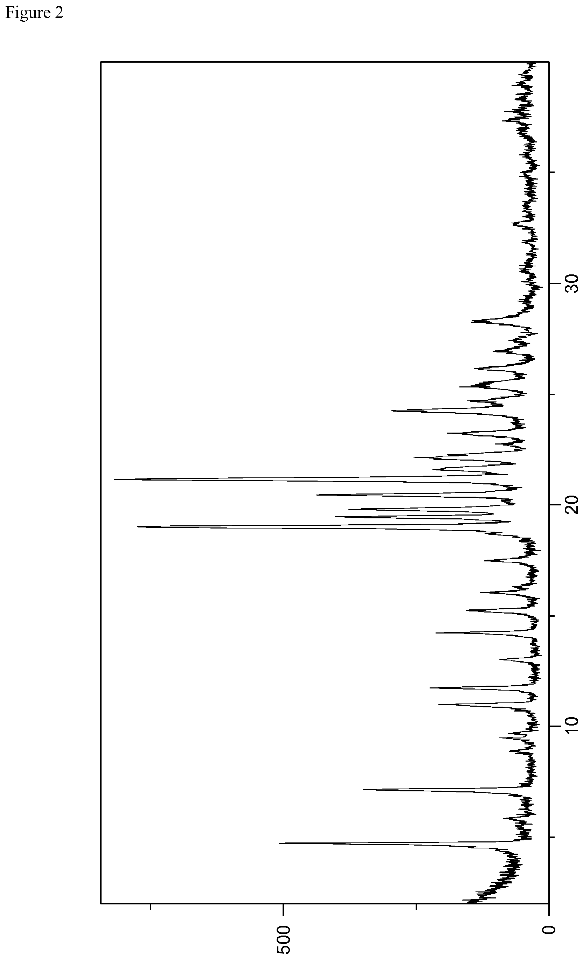

**Figure 2****:** illustrates a representative powder X-ray diffractogram of crystalline *L*-lysine-*d-*amphetamine diadipate form II of the present invention. The x-axis shows the scattering angle in °2-Theta, the y-axis shows the intensity of the scattered X-ray beam in counts of detected photons.

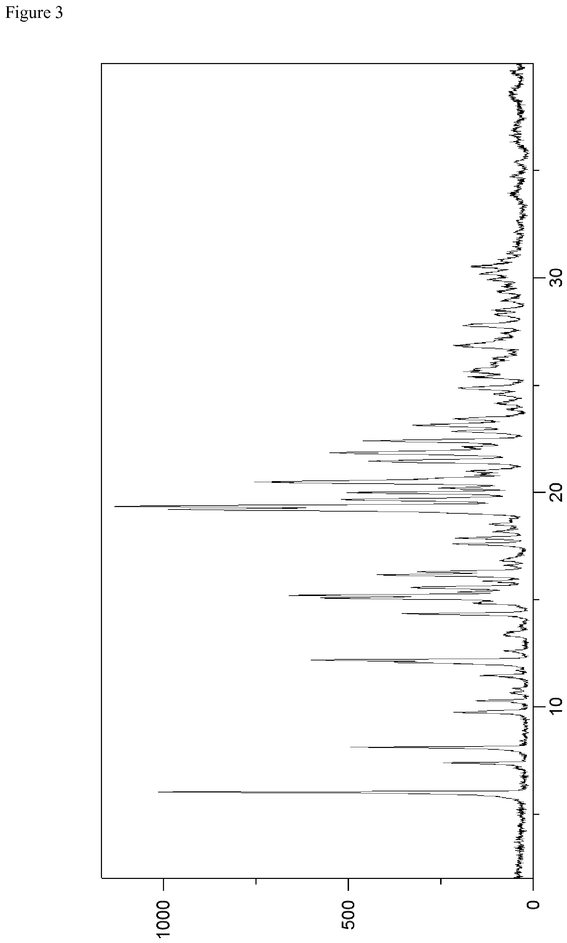

**Figure 3****:** illustrates a representative powder X-ray diffractogram of crystalline *L*-lysine-*d-*amphetamine ditosylate form I of the present invention. The x-axis shows the scattering angle in °2-Theta, the y-axis shows the intensity of the scattered X-ray beam in counts of detected photons.

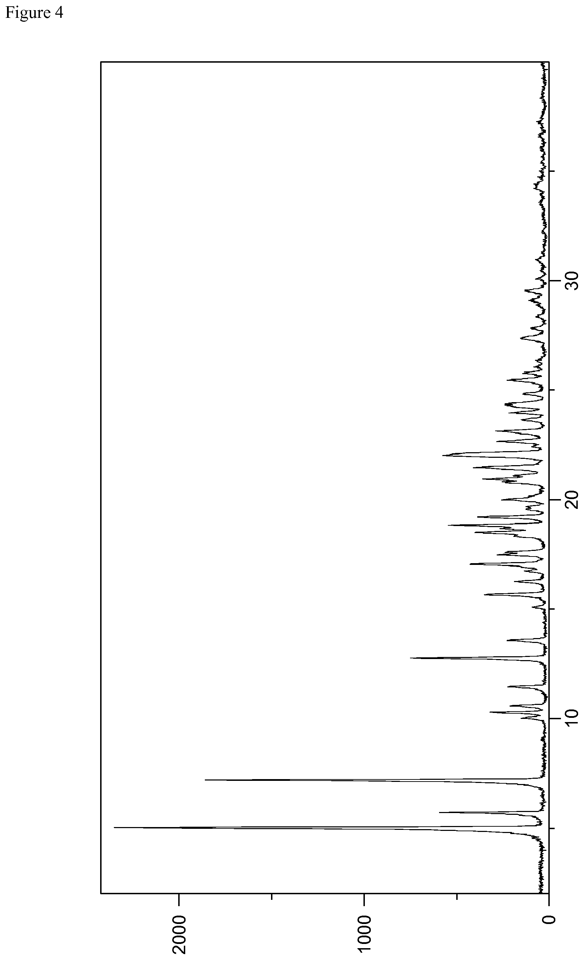

**Figure 4****:** illustrates a representative powder X-ray diffractogram of crystalline *L*-lysine-*d-*amphetamine ditosylate form II of the present invention. The x-axis shows the scattering angle in °2-Theta, the y-axis shows the intensity of the scattered X-ray beam in counts of detected photons.

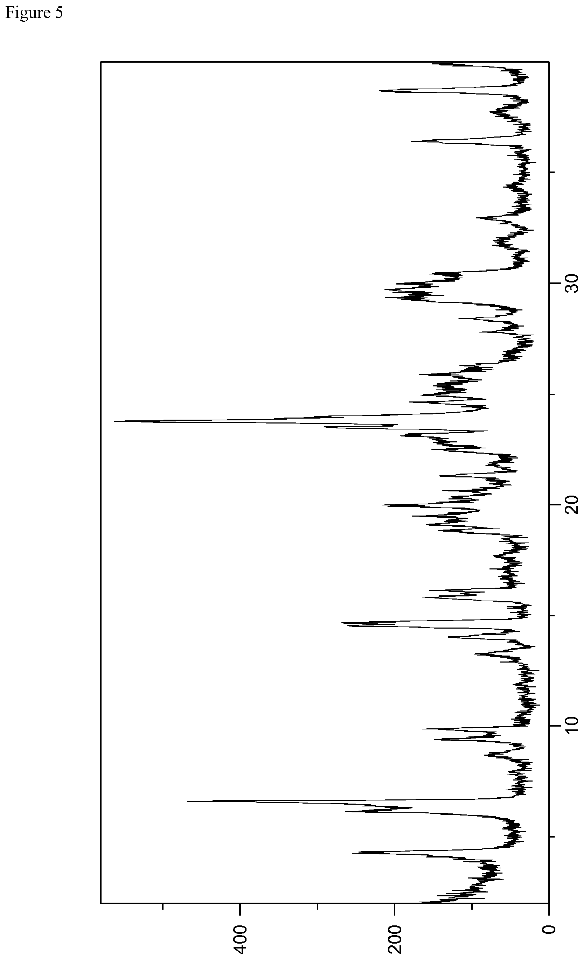

**Figure 5****:** illustrates a representative powder X-ray diffractogram of crystalline *L*-lysine-*d-*amphetamine difumarate of the present invention. The x-axis shows the scattering angle in °2-Theta, the y-axis shows the intensity of the scattered X-ray beam in counts of detected photons.

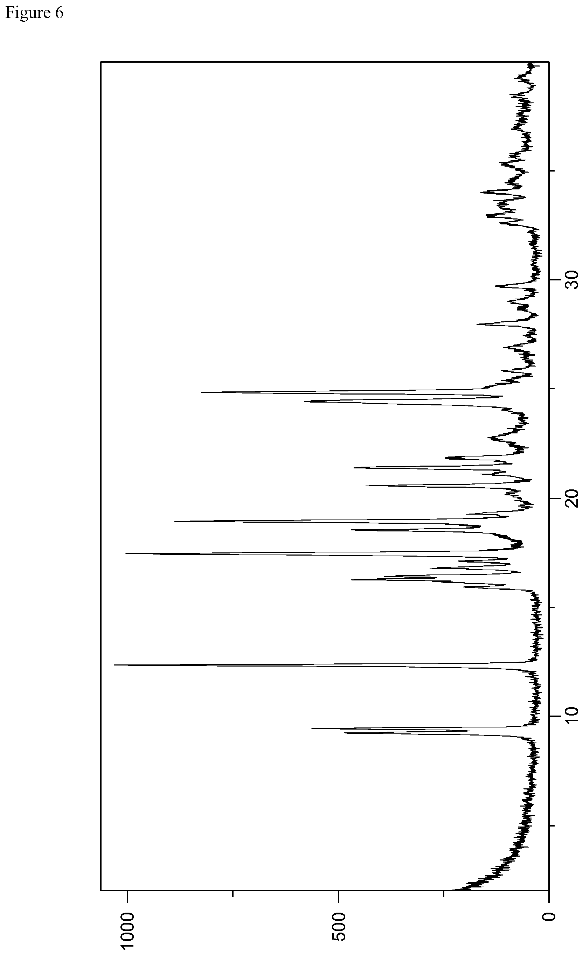

**Figure 6****:** illustrates a representative powder X-ray diffractogram of crystalline *L*-lysine-*d-*amphetamine dioxalate of the present invention. The x-axis shows the scattering angle in °2-Theta, the y-axis shows the intensity of the scattered X-ray beam in counts of detected photons.

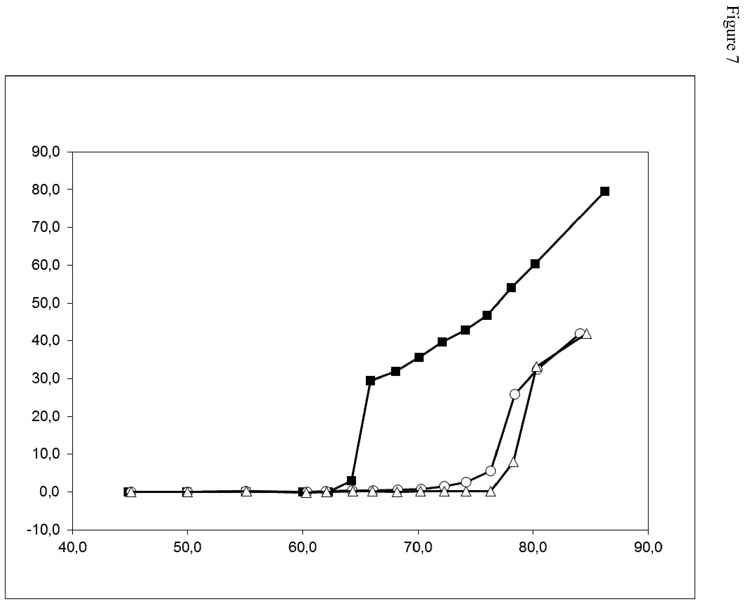

**Figure 7****:** illustrates moisture sorption curves from 45% to 85% relative humidity of *L*-lysine-*d*-amphetamine dimesylate (squares), *L*-lysine-*d*-amphetamine diadipate form I (circles) and *L-*lysine-*d*-amphetamine diadipate form II (triangles). The x-axis displays the relative humidity in percent (%) measured at (25.0 ± 0.1) °C, the y-axis the mass changes in percent (%).

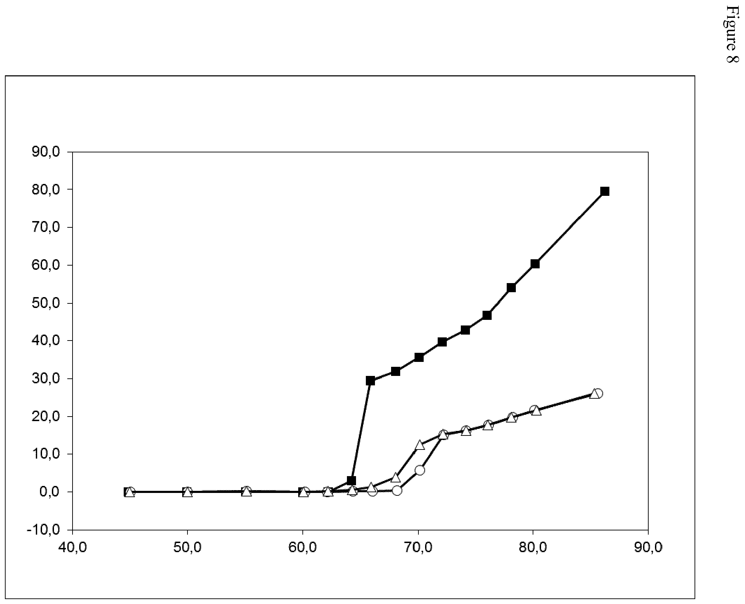

**Figure 8****:** illustrates moisture sorption curves from 45% to 85% relative humidity of *L*-lysine-*d*-amphetamine dimesylate (squares), *L*-lysine-*d*-amphetamine ditosylate form I (circles) and *L*-lysine-*d*-amphetamine ditosylate form II (triangles). The x-axis displays the relative humidity in percent (%) measured at (25.0 ± 0.1) °C, the y-axis the mass changes in percent (%).

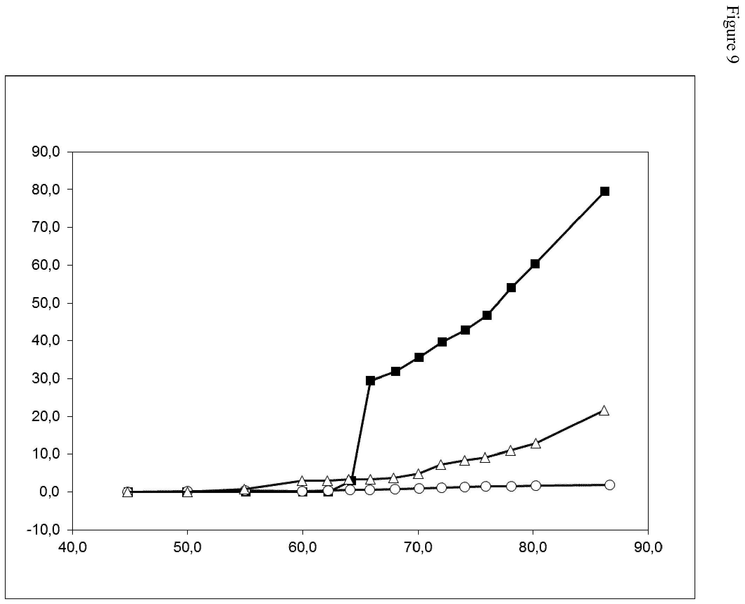

**Figure 9****:** illustrates moisture sorption curves from 45% to 85% relative humidity of *L*-lysine-*d*-amphetamine dimesylate (squares), *L*-lysine-*d*-amphetamine dioxalate (circles) and *L*-lysine-*d*-amphetamine difumarate (triangles). The x-axis displays the relative humidity in percent (%) measured at (25.0 ± 0.1) °C, the y-axis the mass changes in percent (%).

### DETAILED DESCRIPTION OF THE INVENTION

The invention relates to crystalline salts of *L*-lysine-*d*-amphetamine represented by the chemical structure as depicted in formula (II) 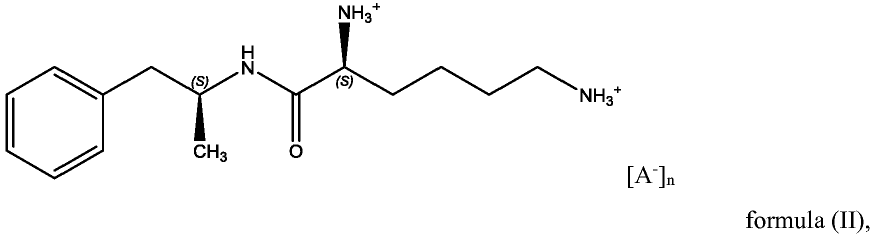 wherein n is in the range of from 1.7 to 2.3, preferably of from 1.8 to 2.2, more preferably of from 1.9 to 2.1, even more preferably of from 1.95 to 2.05 and most preferably n is 2.0 and A is selected from the group consisting of adipic acid, *p*-toluenesulfonic acid, fumaric acid and oxalic acid as defined in the claims.

In one embodiment, the invention relates to crystalline *L*-lysine-*d*-amphetamine diadipate represented by the chemical structure as depicted in formula (IIa) 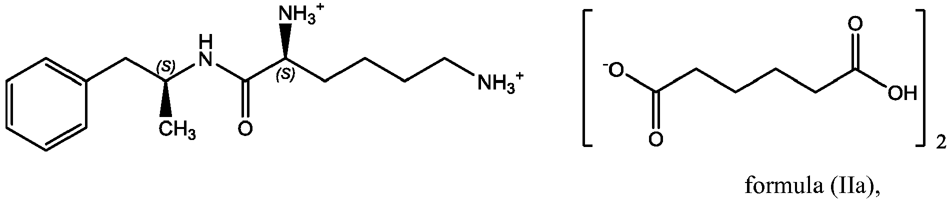 wherein n is in the range of from 1.7 to 2.3, preferably of from 1.8 to 2.2, more preferably of from 1.9 to 2.1, even more preferably of from 1.95 to 2.05 and most preferably n is 2.0.

In another embodiment, the invention relates to crystalline *L*-lysine-*d*-amphetamine ditosylate represented by the chemical structure as depicted in formula (IIb) 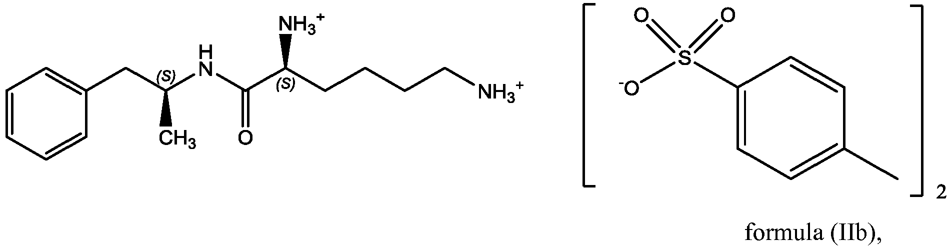 wherein n is in the range of from 1.7 to 2.3, preferably of from 1.8 to 2.2, more preferably of from 1.9 to 2.1, even more preferably of from 1.95 to 2.05 and most preferably n is 2.0.

In yet another embodiment, the invention relates to crystalline *L*-lysine-*d*-amphetamine difumarate represented by the chemical structure as depicted in formula (IIc) 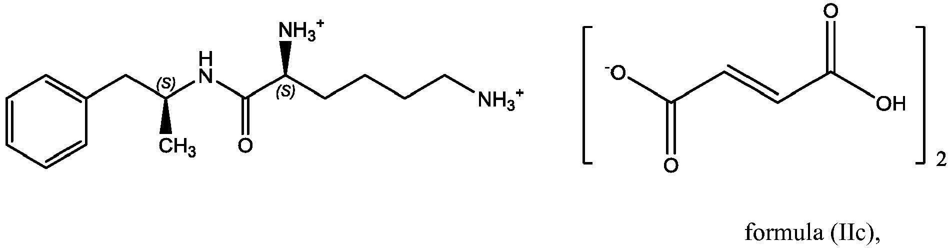 wherein n is in the range of from 1.7 to 2.3, preferably of from 1.8 to 2.2, more preferably of from 1.9 to 2.1, even more preferably of from 1.95 to 2.05 and most preferably n is 2.0.

In still another embodiment, the invention relates to crystalline *L*-lysine-*d*-amphetamine dioxalate represented by the chemical structure as depicted in formula (IId) 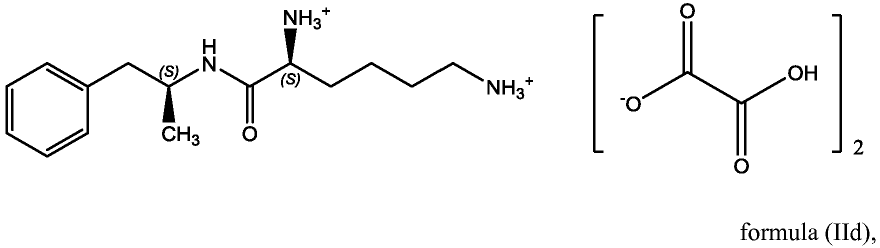 wherein n is in the range of from 1.7 to 2.3, preferably of from 1.8 to 2.2, more preferably of from 1.9 to 2.1, even more preferably of from 1.95 to 2.05 and most preferably n is 2.0.

It was surprisingly found by the present inventors that the crystalline salts of the present invention are less hygroscopic compared to the known *L*-lysine-*d*-amphetamine dimesylate salt. In particular, it was found that the salts of the present invention start to take up water less excessively and only at higher relative humidity levels and do not liquefy as readily as the dimesylate salt does. This is advantageous because less hygroscopic salts facilitate pharmaceutical processing, handling and storage.

The crystalline salts according to the present invention may be characterized by analytical methods well known in the field of the pharmaceutical industry for characterizing solids. A very common method for characterizing crystalline materials and for differentiating one crystalline material from another is for example powder X-ray diffraction. Hence, the crystalline salts of the present invention may be characterized by any one of the following embodiments or by combining two or more of the following embodiments.

### Crystalline L-lysine-d-amphetamine diadipate form I

In one embodiment, the present invention relates to a crystalline form of *L*-lysine-*d-*amphetamine diadipate (form I) characterized by having a powder X-ray diffractogram comprising reflections at 2-Theta angles of:
(4.7 ± 0.2)°, (11.1 ± 0.2)° and (18.9 ± 0.2)°; or
(4.7 ± 0.2)°, (11.1 ± 0.2)°, (14.1 ± 0.2)° and (18.9 ± 0.2)°; or
(4.7 ± 0.2)°, (5.4 ± 0.2)°, (11.1 ± 0.2)°, (14.1 ± 0.2)° and (18.9 ± 0.2)°; or
(4.7 ± 0.2)°, (5.4 ± 0.2)°, (6.5 ± 0.2)°, (11.1 ± 0.2)°, (14.1 ± 0.2)° and (18.9 ± 0.2)°; or
(4.7 ± 0.2)°, (5.4 ± 0.2)°, (6.5 ± 0.2)°, (9.9 ± 0.2)°, (11.1 ± 0.2)°, (14.1 ± 0.2)° and (18.9 ± 0.2)°; or
(4.7 ± 0.2)°, (5.4 ± 0.2)°, (6.5 ± 0.2)°, (9.9 ± 0.2)°, (11.1 ± 0.2)°, (14.1 ± 0.2)°, (18.9 ± 0.2)° and (21.4 ± 0.2)°;
when measured at a temperature in the range of from 20 to 30 °C and a relative humidity in the range of from 0 to 65% with Cu-Kalpha_{1,2} radiation having a wavelength of 0.15419 nm.

In another embodiment, the present invention relates to a crystalline form of *L*-lysine-*d-*amphetamine diadipate (form I) characterized by having a powder X-ray diffractogram essentially the same as shown in figure 1 of the present invention, when measured at a temperature in the range of from 20 to 30 °C and a relative humidity in the range of from 0 to 65% with Cu-Kalpha_{1,2} radiation having a wavelength of 0.15419 nm.

### Crystalline L-lysine-d-amphetamine diadipate form II

In another embodiment, the present invention relates to a crystalline form of *L*-lysine-*d-*amphetamine diadipate (form II) characterized by having a powder X-ray diffractogram comprising reflections at 2-Theta angles of:
(4.7 ± 0.2)°, (7.1 ± 0.2)° and (21.1 ± 0.2)°; or
(4.7 ± 0.2)°, (7.1 ± 0.2)°, (19.0 ± 0.2)° and (21.1 ± 0.2)°; or
(4.7 ± 0.2)°, (7.1 ± 0.2)°, (11.0 ± 0.2)°, (19.0 ± 0.2)° and (21.1 ± 0.2)°; or
(4.7 ± 0.2)°, (7.1 ± 0.2)°, (11.0 ± 0.2)°, (11.7 ± 0.2)°, (19.0 ± 0.2)° and (21.1 ± 0.2)°; or
(4.7 ± 0.2)°, (7.1 ± 0.2)°, (11.0 ± 0.2)°, (11.7 ± 0.2)°, (14.2 ± 0.2)°, (19.0 ± 0.2)° and (21.1 ± 0.2)°; or
(4.7 ± 0.2)°, (7.1 ± 0.2)°, (11.0 ± 0.2)°, (11.7 ± 0.2)°, (14.2 ± 0.2)°, (19.0 ± 0.2)°, (20.4 ± 0.2)° and (21.1 ± 0.2)°;
when measured at a temperature in the range of from 20 to 30 °C and a relative humidity in the range of from 0 to 65% with Cu-Kalpha_{1,2} radiation having a wavelength of 0.15419 nm.

In another embodiment, the present invention relates to a crystalline form of *L*-lysine-*d-*amphetamine diadipate (form II) characterized by having a powder X-ray diffractogram essentially the same as shown in figure 2 of the present invention, when measured at a temperature in the range of from 20 to 30 °C and a relative humidity in the range of from 0 to 65% with Cu-Kalpha_{1,2} radiation having a wavelength of 0.15419 nm.

### Crystalline L-lysine-d-amphetamine ditosylate form I

In one embodiment, the present invention relates to a crystalline form of *L*-lysine-*d-*amphetamine ditosylate (form I) characterized by having a powder X-ray diffractogram comprising reflections at 2-Theta angles of:
(6.0 ± 0.2)°, (7.4 ± 0.2)° and (8.1 ± 0.2)°; or
(6.0 ± 0.2)°, (7.4 ± 0.2)°, (8.1 ± 0.2)° and (12.2 ± 0.2)°; or
(6.0 ± 0.2)°, (7.4 ± 0.2)°, (8.1 ± 0.2)°, (12.2 ± 0.2)° and (15.2 ± 0.2)°; or
(6.0 ± 0.2)°, (7.4 ± 0.2)°, (8.1 ± 0.2)°, (12.2 ± 0.2)°, (14.3 ± 0.2)° and (15.2 ± 0.2)°; or
(6.0 ± 0.2)°, (7.4 ± 0.2)°, (8.1 ± 0.2)°, (9.7 ± 0.2)°, (12.2 ± 0.2)°, (14.3 ± 0.2)° and (15.2 ± 0.2)°; or
(6.0 ± 0.2)°, (7.4 ± 0.2)°, (8.1 ± 0.2)°, (9.7 ± 0.2)°, (12.2 ± 0.2)°, (14.3 ± 0.2)°, (15.2 ± 0.2)° and (19.3 ± 0.2)°;
when measured at a temperature in the range of from 20 to 30 °C and a relative humidity in the range of from 0 to 65% with Cu-Kalpha_{1,2} radiation having a wavelength of 0.15419 nm.

In another embodiment, the present invention relates to a crystalline form of *L*-lysine-*d-*amphetamine ditosylate (form I) characterized by having a powder X-ray diffractogram essentially the same as shown in figure 3 of the present invention, when measured at a temperature in the range of from 20 to 30 °C and a relative humidity in the range of from 0 to 65% with Cu-Kalpha_{1,2} radiation having a wavelength of 0.15419 nm.

### Crystalline L-lysine-d-amphetamine ditosylate form II

In one embodiment, the present invention relates to a crystalline form of *L*-lysine-*d-*amphetamine ditosylate (form II) characterized by having a powder X-ray diffractogram comprising reflections at 2-Theta angles of:
(5.0 ± 0.2)°, (5.7 ± 0.2)° and (7.2 ± 0.2)°; or
(5.0 ± 0.2)°, (5.7 ± 0.2)°, (7.2 ± 0.2)° and (12.7 ± 0.2)°; or
(5.0 ± 0.2)°, (5.7 ± 0.2)°, (7.2 ± 0.2)°, (11.4 ± 0.2)° and (12.7 ± 0.2)°; or
(5.0 ± 0.2)°, (5.7 ± 0.2)°, (7.2 ± 0.2)°, (11.4 ± 0.2)°, (12.7 ± 0.2)° and (22.0 ± 0.2)°; or
(5.0 ± 0.2)°, (5.7 ± 0.2)°, (7.2 ± 0.2)°, (11.4 ± 0.2)°, (12.7 ± 0.2)°, (13.6 ± 0.2)° and (22.0 ± 0.2)°; or
(5.0 ± 0.2)°, (5.7 ± 0.2)°, (7.2 ± 0.2)°, (11.4 ± 0.2)°, (12.7 ± 0.2)°, (13.6 ± 0.2)°, (15.6 ± 0.2)° and (22.0 ± 0.2)°;
when measured at a temperature in the range of from 20 to 30 °C and a relative humidity in the range of from 0 to 65% with Cu-Kalpha_{1,2} radiation having a wavelength of 0.15419 nm.

In another embodiment, the present invention relates to a crystalline form of *L*-lysine-*d-*amphetamine ditosylate (form II) characterized by having a powder X-ray diffractogram essentially the same as shown in figure 4 of the present invention, when measured at a temperature in the range of from 20 to 30 °C and a relative humidity in the range of from 0 to 65% with Cu-Kalpha_{1,2} radiation having a wavelength of 0.15419 nm.

### Crystalline L-lysine-d-amphetamine difumarate

In another embodiment, the present invention relates to a crystalline form of *L*-lysine-*d-*amphetamine difumarate characterized by having a powder X-ray diffractogram comprising reflections at 2-Theta angles of:
(4.3 ± 0.2)°, (6.6 ± 0.2)° and (23.8 ± 0.2)°; or
(4.3 ± 0.2)°, (6.6 ± 0.2)°, (9.4 ± 0.2)° and (23.8 ± 0.2)°; or
(4.3 ± 0.2)°, (6.6 ± 0.2)°, (9.4 ± 0.2)°, (14.7 ± 0.2)° and (23.8 ± 0.2)°; or
(4.3 ± 0.2)°, (6.2 ± 0.2)°, (6.6 ± 0.2)°, (9.4 ± 0.2)°, (14.7 ± 0.2)° and (23.8 ± 0.2)°; or
(4.3 ± 0.2)°, (6.2 ± 0.2)°, (6.6 ± 0.2)°, (9.4 ± 0.2)°, (14.7 ± 0.2)°, (15.8 ± 0.2)° and (23.8 ± 0.2)°; or
(4.3 ± 0.2)°, (6.2 ± 0.2)°, (6.6 ± 0.2)°, (9.4 ± 0.2)°, (14.0 ± 0.2)°, (14.7 ± 0.2)°, (15.8 ± 0.2)° and (23.8 ± 0.2)°;
when measured at a temperature in the range of from 20 to 30 °C and a relative humidity in the range of from 0 to 65% with Cu-Kalpha_{1,2} radiation having a wavelength of 0.15419 nm.

In another embodiment, the present invention relates to a crystalline form of *L*-lysine-*d-*amphetamine difumarate characterized by having a powder X-ray diffractogram essentially the same as shown in figure 5 of the present invention, when measured at a temperature in the range of from 20 to 30 °C and a relative humidity in the range of from 0 to 65% with Cu-Kalpha_{1,2} radiation having a wavelength of 0.15419 nm.

### Crystalline L-lysine-d-amphetamine dioxalate

In another embodiment, the present invention relates to a crystalline form of *L*-lysine-*d-*amphetamine dioxalate characterized by having a powder X-ray diffractogram comprising reflections at 2-Theta angles of:
(9.2 ± 0.2)°, (9.4 ± 0.2)°and (12.3 ± 0.2)°; or
(9.2 ± 0.2)°, (9.4 ± 0.2)°, (12.3 ± 0.2)° and (16.3 ± 0.2)°; or
(9.2 ± 0.2)°, (9.4 ± 0.2)°, (12.3 ± 0.2)°, (16.3 ± 0.2)° and (17.4 ± 0.2)° or
(9.2 ± 0.2)°, (9.4 ± 0.2)°, (12.3 ± 0.2)°, (16.3 ± 0.2)°, (17.4 ± 0.2)° and (18.9 ± 0.2)°; or
(9.2 ± 0.2)°, (9.4 ± 0.2)°, (12.3 ± 0.2)°, (16.3 ± 0.2)°, (17.4 ± 0.2)°, (18.9 ± 0.2)° and (20.6 ± 0.2)°; or
(9.2 ± 0.2)°, (9.4 ± 0.2)°, (12.3 ± 0.2)°, (16.3 ± 0.2)°, (17.4 ± 0.2)°, (18.9 ± 0.2)°, (20.6 ± 0.2)° and (24.8 ± 0.2)°;
when measured at a temperature in the range of from 20 to 30 °C and a relative humidity in the range of from 0 to 65% with Cu-Kalpha_{1,2} radiation having a wavelength of 0.15419 nm.

In another embodiment, the present invention relates to a crystalline form of *L*-lysine-*d-*amphetamine dioxalate characterized by having a powder X-ray diffractogram essentially the same as shown in figure 6 of the present invention, when measured at a temperature in the range of from 20 to 30 °C and a relative humidity in the range of from 0 to 65% with Cu-Kalpha_{1,2} radiation having a wavelength of 0.15419 nm.

It was surprisingly found by the present inventors that the salts of the present invention are less hygroscopic compared to the *L*-lysine-*d*-amphetamine dimesylate salt. In particular, it was found that the salts of the present invention take up water less excessively and only at higher relative humidity levels. Consequently, deliquescence does not occure as readily as observed for the dimesylate salt (see example 8 as well as figures 7 to 9 herein). Said properties favour the salts of the present invention for pharmaceutical processing, handling and storage. It is worthwile mentioning that also, WO 2006/121552 A2, paragraph [099], acknowledges that less hygroscopic salts facilitate handling.

Hence, in a further aspect the present invention relates to the use of the crystalline *L*-lysine-*d-*amphetamine salts of the present invention as defined in any one of the embodiments described above for the preparation of a pharmaceutical composition.

In yet a further aspect, the present invention relates to a pharmaceutical composition comprising one or more of the crystalline *L*-lysine-*d*-amphetamine salts of the present invention as defined in any one of the embodiments described above, preferably in an effective and/or predetermined amount, and at least one pharmaceutically acceptable excipient.

Preferably, the predetermined and/or effective amount of the one or more crystalline *L*-lysine-*d*-amphetamine salts of the present invention is in the range of from 10 to 250 mg, preferably of from 20 to 70 mg calculated as *L*-lysine-*d*-amphetamine. For example the predetermined and/or effective amount of the one or more crystalline *L*-lysine-*d*-amphetamine salts of the present invention is 20 mg, 30 mg, 40 mg, 50 mg, 60 mg or 70 mg, preferably 30 mg, 50 mg or 70 mg calculated as *L*-lysine-*d*-amphetamine.

The at least one pharmaceutically acceptable excipient, which is comprised in the pharmaceutical composition of the present invention is preferably selected from the group consisting of fillers/diluents, disintegrants and lubricants. Preferably, all of these pharmaceutically acceptable excipients are comprised by the pharmaceutical composition of the present invention. More preferably, the filler/diluent is microcrystalline cellulose (e.g. Avicel® PH-102), the disintegrant is croscarmellose sodium and the lubricant is magnesium stearate. It should be appreciated that an excipient may have more than one function e.g. microcrystalline cellulose may act contemporaneously as filler and disintegrant.

Preferably, the pharmaceutical composition of the present invention as described above is an oral solid dosage form.

In a particular preferred embodiment, the pharmaceutical composition of the present invention as describe above is a capsule. In a further embodiment, the capsule shell is a gelatin shell or a hydroxypropylmethylcellulose (HPMC) shell.

A capsule of the present invention can be prepared by mixing one or more of the crystalline *L-*lysine-*d*-amphetamine salts of the present invention as defined in any one of the embodiments described above with microcrystalline cellulose, croscarmellose sodium and magnesium stearate to form a uniform blend. Optionally, one or more of the components may be subjected to a particle size reduction step such as milling and/or sieving before blending. The blend is then filled into capsules e.g. hard gelatin capsules.

In a further aspect, the present invention relates to the crystalline *L*-lysine-*d*-amphetamine salts of the present invention or the pharmaceutical composition comprising one or more of the crystalline *L*-lysine-*d*-amphetamine salts of the present invention as defined in any one of the above described aspects and their corresponding embodiments for use as a medicament.

In yet another aspect, the present invention relates to the crystalline *L*-lysine-*d*-amphetamine salts of the present invention or the pharmaceutical composition comprising one or more of the crystalline *L*-lysine-*d*-amphetamine salts of the present invention as defined in any one of the above described aspects and their corresponding embodiments for use in the treatment of Attention Deficit and Hyperactivity Disorder (ADHD) and Binge Eating Disorder (BED) symptoms.

### EXAMPLES

### Powder X-ray diffraction

Powder X-ray diffraction was performed with a PANalytical X'Pert PRO diffractometer equipped with a theta/theta coupled goniometer in transmission geometry, Cu-Kalpha_{1,2} radiation (wavelength 0.15419 nm) with a focusing mirror and a solid state PIXcel detector. Diffractograms were recorded at a tube voltage of 45 kV and a tube current of 40 mA, applying a stepsize of 0.013° 2-Theta with 40s per step (255 channels) in the angular range of 2° to 40° 2-Theta at ambient conditions. A typical precision of the 2-Theta values is in the range of ± 0.2° 2-Theta. Thus, the diffraction peak of *L*-lysine-*d*-amphetamine diadipate form I that appears for example at 4.7° 2-Theta can appear in the range of from 4.5 to 4.9° 2-Theta on most X-ray diffractometers under standard conditions.

The following non-limiting examples are illustrative for the disclosure and are not to be construed as to be in any way limiting for the scope of the invention.

### Example 1: Preparation of L-lysine-d-amphetamine free base from L-lysine-d-amphetamine dimesylate

*L*-lysine-*d*-amphetamine dimesylate (10.0 g, e.g. prepared according to example 2b of WO 2006/121552 A2) was dissolved in a mixture of water (10 mL), dichloromethane (50 mL) and aqueous NaOH solution (10 mL, 5M). After 5 minutes of heavy stirring the layers were separated and the aqueous layer was extracted twice with dichloromethane (2 x 25 mL). The collected organic layers were dried over sodium sulfate and filtrated before the solvent was removed on a rotary evaporator (40 °C, final vacuum 40 mbar) yielding *L*-lysine-*d-*amphetamine free base as colorless oil.

### Example 2: Preparation of crystalline L-lysine-d-amphetamine diadipate (form I)

*L*-lysine-*d*-amphetamine free base (1.25 g, prepared analogously as described in example 1 herein) was dissolved in ethanol (12.5 mL). A hot solution of adipic acid (1.42 g) in ethanol (15 mL) was added and the obtained clear solution was stirred at room temperature. After 1 hour of stirring *L*-lysine-*d*-amphetamine diadipate form I seed crystals (10 mg) were added causing immediate precipitation of a crystalline solid. The obtained suspension was cooled to 0 °C and further stirred at this temperature for 2 hours. Finally, the crystals were collected by filtration and sucked dry on the filter.
Yield: 1.6 g (62% of theory)

The seed crystals, which have been used in the above described process were prepared by dissolving *L*-lysine-*d*-amphetamine diadipate form II (50 mg, e.g. prepared according to example 3 herein) in ethanol (1 mL) upon heating. After cooling the obtained solution to room temperature diisopropyl ether (0.5 mL) was added until a thin suspension was obtained. Finally, the *L*-lysine-*d*-amphetamine diadipate form I crystals were collected by filtration.

A representative diffractogram of *L*-lysine-*d*-amphetamine diadipate form I is displayed in figure 1 herein. The corresponding reflection list is provided in table 1 below.

**Table 1: Reflection positions of L-lysine-d-amphetamine diadipate form 1 in the range of from 2 to 30° 2-Theta; A typical precision of the 2-Theta values is in the range of ± 0.2° 2-Theta;**

| **Reflection position [° 2-Theta**] | **Reflection position [° 2-Theta**] |
|---|---|
| 4.7 | 19.1 |
| 5.4 | 19.7 |
| 6.5 | 20.8 |
| 7.5 | 21.4 |
| 9.4 | 22.0 |
| 9.9 | 22.5 |
| 11.1 | 23.8 |
| 14.1 | 25.7 |
| 16.0 | 26.5 |
| 18.9 | 27.5 |

### Example 3: Preparation of L-lysine-d-amphetamine diadipate form II

*L*-lysine-*d*-amphetamine free base (5.87 g, prepared analogously as described in example 1 herein) was dissolved in methanol (30 mL). A mixture of adipic acid (6.4 g) in methanol (30 mL) was added and the mixture was heated to 60 °C in order to obtain a clear solution. After cooling the solution to about 30 °C diisopropyl ether (40 mL) and *L*-lysine-*d*-amphetamine diadipate form II seeds were added subsequently. The mixture was stirred at 30 °C for approximately 20 hours, whereat a homogenous suspension was obtained. Additional diisopropyl ether (60 mL) was added within 4 hours before the suspension was cooled to 0 °C and stirred for 24 hours at this temperature. Finally, the crystals were collected by filtration and dried at room temperature under vaccum (30 mbar).
Yield: 11.2g (91% of theory)

The seed crystals, which have been used in the above described process were prepared by reacting *L*-lysine-*d*-amphetamine free base (100 mg, prepared analogously as described in example 1 herein) with adipic acid (111 mg) in methanol (1 mL) and allowing the solvent to evaporate at room temperature. The residue was dissolved in ethanol (1 mL) upon heating before the obtained solution was cooled to room temperature in order to initate crystallization. Finally, the *L*-lysine-*d*-amphetamine diadipate form II crystals were collected by filtration.

A representative diffractogram of crystalline form II of *L*-lysine-*d*-amphetamine diadipate is displayed in figure 2 herein. The corresponding reflection list is provided in table 2 below.

**Table 2: Reflection positions of L-lysine-d-amphetamine diadipate in the range of from 2 to 30° 2-Theta; A typical precision of the 2-Theta values is in the range of ± 0.2° 2-Theta;**

| **Reflection position [° 2-Theta**] | **Reflection position [° 2-Theta**] | **Reflection position [° 2-Theta**] |
|---|---|---|
| 4.7 | 15.2 | 22.1 |
| 5.8 | 16.0 | 22.7 |
| 7.1 | 16.3 | 23.2 |
| 8.9 | 17.5 | 24.2 |
| 9.5 | 19.0 | 24.7 |
| 9.7 | 19.4 | 25.4 |
| 11.0 | 19.8 | 26.1 |
| 11.7 | 20.4 | 26.9 |
| 13.0 | 21.1 | 27.4 |
| 14.2 | 21.6 | 28.3 |

### Example 4: Preparation of L-lysine-d-amphetamine ditosylate form I

*L*-lysine-*d*-amphetamine free base (4.4 g, prepared analogously as described in example 1 herein) was dissolved in ethanol (33 mL). A solution of *p*-toluene sulfonic acid monohydrate (6.5 g) in ethanol (30 mL) and *L*-lysine-*d*-amphetamine ditosylate form I seed crystals (10 mg) were added subsequently and the mixture was stirred at room temperature for 15 min. Then diisopropyl ether (100 mL) and again *L*-lysine-*d*-amphetamine ditosylate form I seed crystals (10 mg) were added, whereupon crystallization of a crystalline solid occured. After 17 hours of stirring diisopropyl ether (100 mL) was added and the suspension was stirred for another 22.5 hours at room temperature. Finally, the crystals were collected by filtration and dried at room temperature under vaccum (30 mbar) for 16 hours.
Yield: 7.6 g (57% of theory)

The seed crystals, which have been used in the above described process were prepared by reacting *L*-lysine-*d*-amphetamine free base (100 mg, prepared analogously as described in example 1 herein) with *p*-toluenesulfonic acid monohydrate (145 mg) in methanol (1 mL) and allowing the solvent to evaporate at room temperature. The residue was dissolved in ethanol (1 mL) upon heating before the obtained solution was cooled to room temperature and diisopropyl ether (0.5 mL) was added in order to initate crystallization. Finally, the *L*-lysine-*d*-amphetamine ditosylate form I crystals were collected by filtration.

A representative diffractogram of crystalline *L*-lysine-*d*-amphetamine ditosylate form I is displayed in figure 3 herein. The corresponding reflection list is provided in table 3 below.

**Table 3: Reflection positions of L-lysine-d-amphetamine ditosylate form I in the range of from 2 to 30° 2-Theta; A typical precision of the 2-Theta values is in the range of ± 0.2° 2-Theta;**

| **Reflection position [° 2-Theta**] | **Reflection position [° 2-Theta**] | **Reflection position [° 2-Theta**] | **Reflection position [° 2-Theta**] |
|---|---|---|---|
| 6.0 | 15.0 | 19.3 | 23.9 |
| 7.4 | 15.2 | 19.7 | 24.1 |
| 8.1 | 15.3 | 20.0 | 24.6 |
| 9.7 | 15.5 | 20.2 | 24.9 |
| 10.3 | 15.8 | 20.4 | 25.4 |
| 10.6 | 16.1 | 20.5 | 25.7 |
| 10.8 | 16.3 | 21.0 | 26.2 |
| 11.5 | 16.6 | 21.4 | 26.8 |
| 12.1 | 16.8 | 21.8 | 27.8 |
| 12.2 | 17.6 | 22.1 | 28.3 |
| 12.6 | 17.9 | 22.4 | 28.5 |
| 13.4 | 18.2 | 22.8 | 28.9 |
| 14.3 | 18.5 | 23.1 | 29.3 |
| 14.8 | 19.2 | 23.4 | 29.9 |

### Example 5: Preparation of L-lysine-d-amphetamine ditosylate form II

*L*-lysine-*d*-amphetamine ditosylate form I (1.0 g, prepared according to example 4 herein) was stored in a drying chamber at a temperature of 120-130 °C for 9 days to obtain *L*-lysine-*d-*amphetamine ditosylate form II quantitatively.

A representative diffractogram of *L*-lysine-*d*-amphetamine ditosylate form II is displayed in figure 4 herein. The corresponding reflection list is provided in table 4 below.

**Table 4: Reflection positions of L-lysine-d-amphetamine ditosylate form II in the range of from 2 to 30° 2-Theta; A typical precision of the 2-Theta values is in the range of ± 0.2° 2-Theta;**

| **Reflection position [° 2-Theta**] | **Reflection position [° 2-Theta**] | **Reflection position [° 2-Theta**] | **Reflection position [° 2-Theta**] |
|---|---|---|---|
| 5.0 | 16.2 | 20.8 | 24.3 |
| 5.7 | 16.7 | 20.9 | 24.8 |
| 7.2 | 17.1 | 21.1 | 25.5 |
| 10.0 | 17.5 | 21.5 | 25.8 |
| 10.3 | 17.6 | 22.0 | 26.3 |
| 10.6 | 18.3 | 22.1 | 27.3 |
| 11.4 | 18.5 | 22.7 | 27.8 |
| 12.7 | 18.8 | 23.1 | 28.4 |
| 13.6 | 19.2 | 23.6 | 29.1 |
| 15.1 | 19.6 | 23.9 | 29.5 |
| 15.6 | 20.0 | 24.2 | |

### Example 6: Preparation of crystalline L-lysine-d-amphetamine difumarate

A suspension of *L*-lysine-*d*-amphetamine dimesylate (1 g, e.g. prepared according to example 2b of WO 2006/121552 A2) and sodium ethoxide (0.3 g, assay >95%) in ethanol (10 mL) was stirred at room temperature for 60 min before the remaining solid portion was removed by filtration. Fumaric acid (0.51 g) was added and dissolved with the aid of an ultrasonic bath. The obtained solution was stirred at room temperature for 16 hours, whereupon a suspension was obtained. Diisopropyl ether (10 mL) was added and the suspension was stirred for another 1 hour. Finally, the crystals were collected by filtration and dried at room temperature under vaccum (30 mbar) for 16 hours.

A representative diffractogram of crystalline *L*-lysine-*d*-amphetamine difumarate is displayed in figure 5 herein. The corresponding reflection list is provided in table 5 below.

**Table 5: Reflection positions of crystalline L-lysine-d-ampetamine difumarate in the range of from 2 to 30° 2-Theta; A typical precision of the 2-Theta values is in the range of ± 0.2° 2-Theta;**

| **Reflection position [° 2-Theta**] | **Reflection position [° 2-Theta**] | **Reflection position [° 2-Theta**] | **Reflection position [° 2-Theta**] |
|---|---|---|---|
| 4.3 | 14.7 | 20.6 | 25.4 |
| 6.2 | 15.8 | 21.3 | 25.9 |
| 6.6 | 16.1 | 22.5 | 26.2 |
| 8.7 | 17.7 | 23.1 | 27.8 |
| 9.4 | 18.8 | 23.5 | 28.4 |
| 9.9 | 19.1 | 23.8 | 29.3 |
| 13.2 | 19.5 | 24.0 | 29.7 |
| 14.0 | 20.0 | 24.6 | |
| 14.5 | 20.3 | 25.0 | |

### Example 7: Preparation of crystalline L-lysine-d-amphetamine dioxalate

*L*-lysine-*d*-amphetamine free base (4.4 g, prepared analogously as described in example 1 herein) was dissolved in ethanol (33 mL). A solution of oxalic acid (3.1 g) in ethanol (25 mL) was added resulting in a very thick suspension. The suspension was diluted with ethanol (100 mL) and water (5 mL), warmed to 65 °C and stirred at this temperature for 0.5 hours. Thereafter, diisopropyl ether (100 mL) was added and the suspension was cooled to room temperature and stirred for 2 hours. Finally, the crystals were collected by filtration and dried at room temperature under vaccum (30 mbar) for 16 hours.
Yield: 5.7 g (59% of theory)

A representative diffractogram of crystalline *L*-lysine-*d*-amphetamine dioxalate is displayed in figure 6 herein. The corresponding reflection list is provided in table 6 below.

**Table 6: Reflection positions of crystalline L-lysine-d-amphetamine dioxalate in the range of from 2 to 30° 2-Theta; A typical precision of the 2-Theta values is in the range of ± 0.2° 2-Theta;**

| **Reflection position [° 2-Theta**] | **Reflection position [° 2-Theta**] | **Reflection position [° 2-Theta**] |
|---|---|---|
| 9.2 | 18.5 | 24.8 |
| 9.4 | 18.9 | 25.8 |
| 12.3 | 19.3 | 26.9 |
| 15.9 | 20.6 | 28.0 |
| 16.3 | 21.1 | 28.7 |
| 16.4 | 21.4 | 29.0 |
| 16.8 | 21.8 | 29.7 |
| 17.1 | 22.7 | |
| 17.4 | 24.5 | |

### Example 8: Gravimetric moisture sorption

Moisture sorption isotherms of all samples were recorded with an SPSx-1µ moisture sorption analyzer (ProUmid, Ulm) at a temperature of (25.0 ± 0.1) °C. The measurement cycle was started at 45% relative humidity. Relative humidity was then increased to 60% in 5% steps, followed by a further increase to 80% in 2% steps and a final increase to approximately 85%. Samples were kept at each humidity step for approximately 6 hours. At the end of this period pictures of the samples were taken at each humidity step with a camera directly connected to the instrument. Figures 7 to 9 of the present invention show the mass changes (Δm in % on the y-axis) of the respective solid-state forms at the end of each humidity step during the sorption cycle from 45% to 85% relative humidity (on the x-axis). The sample weights at 45% relative humidity were set as starting values (Δm = 0).

As apparent from figures 7 to 9 the dimesylate salt of *L*-lysine-*d*-amphetamine starts to take up water from the surrounding atmosphere significantly earlier (at lower relative humidity levels) and to a significant higher extent compared to the solid-state forms of *L*-lysine-*d*-amphetamine of the present invention. This tremendous water uptake is connected with deliquescence, which occured already at 66% relative humidity for the dimesylate salt. Deliquescence also occurred in most solid-state forms of the present invention, but only at significantly higher relative humidity levels (see table 7).

**Table 7: Deliquescence observed for the different solid-state forms during moisture sorption experiments**

| Solid-state form of *L*-lysine-*d*-amphetamine | Full deliquescence observed at |
|---|---|
| dimesylate disclosed in WO 2005/000334 A1 | 66% |
| diadipate form 1 of present invention | 78% |
| diadipate form 2 of present invention | 80% |
| ditosylate form 1 of present invention | 70% |
| ditosylate form 2 of present invention | 68% |
| difumarate of present invention | 80% |
| dioxalate of present invention | no deliquescence observed |

## Claims

1. A crystalline salt of *L*-lysine-*d*-amphetamine represented by the chemical structure as depicted in formula (II) 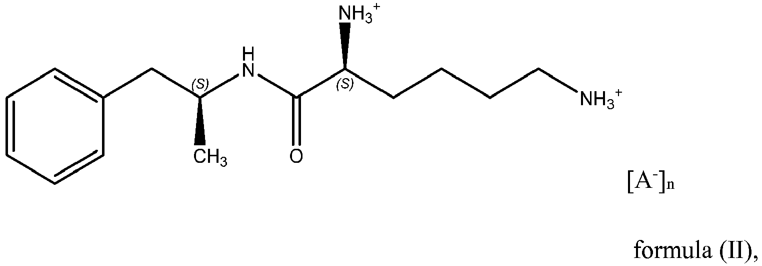 wherein n is in the range of from 1.9 to 2.1 and A is selected from the group consisting of adipic acid, *p*-toluene sulfonic acid, fumaric acid and oxalic acid, and wherein the crystalline salt ist selected from the group consisting of
• *L*-lysine-*d*-amphetamine diadipate (form I), **characterized by** having a powder X-ray diffractogram comprising reflections at 2-Theta angles of (4.7 ± 0.2)°, (11.1 ± 0.2)° and (18.9 ± 0.2)°, when measured at a temperature in the range of from 20 to 30 °C and a relative humidity in the range of from 0 to 65% with Cu-Kalpha_{1,2} radiation having a wavelength of 0.15419 nm;
• *L*-lysine-*d*-amphetamine diadipate (form II), **characterized by** having a powder X-ray diffractogram comprising reflections at 2-Theta angles of (4.7 ± 0.2)°, (7.1 ± 0.2)° and (21.1 ± 0.2)°, when measured at a temperature in the range of from 20 to 30 °C and a relative humidity in the range of from 0 to 65% with Cu-Kalpha_{1,2} radiation having a wavelength of 0.15419 nm;
• *L*-lysine-*d*-amphetamine ditosylate (form I), **characterized by** having a powder X-ray diffractogram comprising reflections at 2-Theta angles of (6.0 ± 0.2)°, (7.4 ± 0.2)° and (8.1 ± 0.2)°, when measured at a temperature in the range of from 20 to 30 °C and a relative humidity in the range of from 0 to 65% with Cu-Kalpha_{1,2} radiation having a wavelength of 0.15419 nm;
• *L*-lysine-*d*-amphetamine ditosylate (form II), **characterized by** having a powder X-ray diffractogram comprising reflections at 2-Theta angles of (5.0 ± 0.2)°, (5.7 ± 0.2)° and (7.2 ± 0.2)°, when measured at a temperature in the range of from 20 to 30 °C and a relative humidity in the range of from 0 to 65% with Cu-Kalpha_{1,2} radiation having a wavelength of 0.15419 nm;
• *L*-lysine-*d*-amphetamine difumarate, **characterized by** having a powder X-ray diffractogram comprising reflections at 2-Theta angles of (4.3 ± 0.2)°, (6.6 ± 0.2)° and (23.8 ± 0.2)°, when measured at a temperature in the range of from 20 to 30 °C and a relative humidity in the range of from 0 to 65% with Cu-Kalpha_{1,2} radiation having a wavelength of 0.15419 nm; and
• *L*-lysine-*d*-amphetamine dioxalate, **characterized by** having a powder X-ray diffractogram comprising reflections at 2-Theta angles of (9.2 ± 0.2)°, (9.4 ± 0.2)°and (12.3 ± 0.2)°, when measured at a temperature in the range of from 20 to 30 °C and a relative humidity in the range of from 0 to 65% with Cu-Kalpha_{1,2} radiation having a wavelength of 0.15419 nm.

2. Use of the crystalline salt as defined in claim 1 for the preparation of a pharmaceutical composition.

3. A pharmaceutical composition comprising the crystalline salt as defined in claim 1 and at least one pharmaceutically acceptable excipient.

4. The pharmaceutical composition of claim 3, wherein the at least one pharmaceutically acceptable excipient is selected from the group consisting of microcrystalline cellulose, croscarmellose sodium and magnesium stearate.

5. The pharmaceutical composition of claim 3 or 4, wherein the pharmaceutical composition is an oral solid dosage form.

6. The pharmaceutical composition according to claim 5, wherein the oral solid dosage form is a capsule.

7. The pharmaceutical composition according to claim 6, wherein the capsule is a hard gelatin capsule

8. The crystalline salt as defined in claim 1 or the pharmaceutical composition according to any one of claims 3 to 7 for use as a medicament.

9. The crystalline salt as defined in claim 1 or the pharmaceutical composition according to any one of claims 3 to 7 for use in the treatment of Attention Deficit and Hyperactivity Disorder (ADHD) and Binge Eating Disorder (BED) symptoms.

## Patentansprüche

1. Kristallines Salz von *L*-Lysin-*d*-amphetamin, das durch die chemische Struktur repräsentiert wird, wie in Formel (II) dargestellt 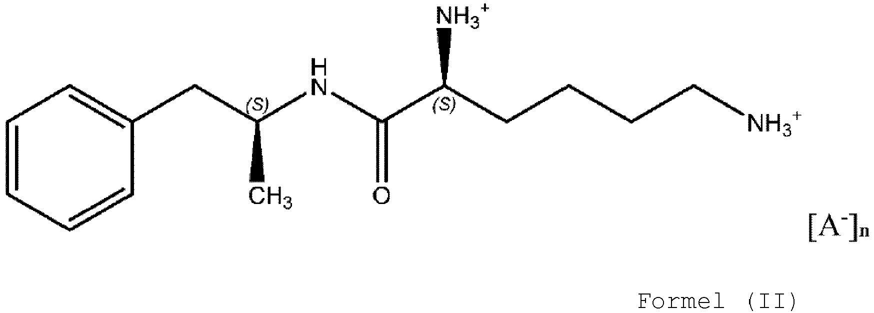 wobei n im Bereich von 1,9 bis 2,1 liegt und A ausgewählt ist aus der Gruppe bestehend aus Adipinsäure, *p*-Toluolsulfonsäure, Fumarsäure und Oxalsäure, und wobei das kristalline Salz ausgewählt ist aus der Gruppe bestehend aus
• *L*-Lysin-*d*-amphetamindiadipat (Form I), **dadurch gekennzeichnet, dass** es ein Pulverröntgendiffraktogramm aufweist, das gemessen bei einer Temperatur im Bereich von 20 bis 30 °C und einer relativen Feuchtigkeit im Bereich von 0 bis 65 % mit Cu-Kalpha_{1,2}-Strahlung mit einer Wellenlänge von 0,15419 nm Reflexionen bei 2-Theta-Winkeln von (4,7 ± 0,2)°, (11,1 ± 0,2)° und (18,9 ± 0,2)° aufweist;
• *L*-Lysin-*d*-amphetamindiadipat (Form II), **dadurch gekennzeichnet, dass** es ein Pulverröntgendiffraktogramm aufweist, das gemessen bei einer Temperatur im Bereich von 20 bis 30 °C und einer relativen Feuchtigkeit im Bereich von 0 bis 65 % mit Cu-Kalpha_{1,2}-Strahlung mit einer Wellenlänge von 0,15419 nm Reflexionen bei 2-Theta-Winkeln von (4,7 ± 0,2)°, (7,1 ± 0,2)° und (21,1 ± 0,2)° aufweist;
• *L*-Lysin-*d*-amphetaminditosylat (Form I), **dadurch gekennzeichnet, dass** es ein Pulverröntgendiffraktogramm aufweist, das gemessen bei einer Temperatur im Bereich von 20 bis 30 °C und einer relativen Feuchtigkeit im Bereich von 0 bis 65 % mit Cu-Kalpha_{1,2}-Strahlung mit einer Wellenlänge von 0,15419 nm Reflexionen bei 2-Theta-Winkeln von (6,0 ± 0,2)°, (7,4 ± 0,2)° und (8,1 ± 0,2)° aufweist;
• *L*-Lysin-*d*-amphetaminditosylat (Form II), **dadurch gekennzeichnet, dass** es ein Pulverröntgendiffraktogramm aufweist, das gemessen bei einer Temperatur im Bereich von 20 bis 30 °C und einer relativen Feuchtigkeit im Bereich von 0 bis 65 % mit Cu-Kalpha_{1,2}-Strahlung mit einer Wellenlänge von 0,15419 nm Reflexionen bei 2-Theta-Winkeln von (5,0 ± 0,2)°, (5,7 ± 0,2)° und (7,2 ± 0,2)° aufweist;
• *L*-Lysin-*d*-amphetamindifumarat, **dadurch gekennzeichnet, dass** es ein Pulverröntgendiffraktogramm aufweist, das gemessen bei einer Temperatur im Bereich von 20 bis 30 °C und einer relativen Feuchtigkeit im Bereich von 0 bis 65 % mit Cu-Kalpha_{1,2}-Strahlung mit einer Wellenlänge von 0,15419 nm Reflexionen bei 2-Theta-Winkeln von (4,3 ± 0,2)°, (6,6 ± 0,2)° und (23,8 ± 0,2)° aufweist; und
• *L*-Lysin-*d*-amphetamindioxalat, **dadurch gekennzeichnet, dass** es ein Pulverröntgendiffraktogramm aufweist, das gemessen bei einer Temperatur im Bereich von 20 bis 30 °C und einer relativen Feuchtigkeit im Bereich von 0 bis 65 % mit Cu-Kalpha_{1,2}-Strahlung mit einer Wellenlänge von 0,15419 nm Reflexionen bei 2-Theta-Winkeln von (9,2 ± 0,2)°, (9,4 ± 0,2)° und (12,3 ± 0,2)° aufweist.

2. Verwendung des kristallinen Salzes nach Anspruch 1 zur Herstellung einer pharmazeutischen Zusammensetzung.

3. Pharmazeutische Zusammensetzung, umfassend das kristalline Salz nach Anspruch 1 und mindestens einen pharmazeutisch verträglichen Hilfsstoff.

4. Pharmazeutische Zusammensetzung nach Anspruch 3, wobei der mindestens eine pharmazeutisch verträgliche Hilfsstoff ausgewählt ist aus der Gruppe bestehend aus mikrokristalliner Cellulose, Croscarmellose-Natrium und Magnesiumstearat.

5. Pharmazeutische Zusammensetzung nach Anspruch 3 oder 4, wobei die pharmazeutische Zusammensetzung eine orale feste Darreichungsform ist.

6. Pharmazeutische Zusammensetzung nach Anspruch 5, wobei die orale feste Darreichungsform eine Kapsel ist.

7. Pharmazeutische Zusammensetzung nach Anspruch 6, wobei die Kapsel eine Hartgelatinkapsel ist.

8. Kristallines Salz nach Anspruch 1 oder pharmazeutische Zusammensetzung nach einem der Ansprüche 3 bis 7 zur Verwendung als Medikament.

9. Kristallines Salz nach Anspruch 1 oder pharmazeutische Zusammensetzung nach einem der Ansprüche 3 bis 7 zur Verwendung bei der Behandlung von Symptomen von Aufmerksamkeitsdefizit- und Hyperaktivitätsstörung (ADHS) und Binge Eating Disorder (BED).

## Revendications

1. Sel cristallin de *L*-lysine-*d*-amphétamine représenté par la structure chimique décrite dans la formule (II) 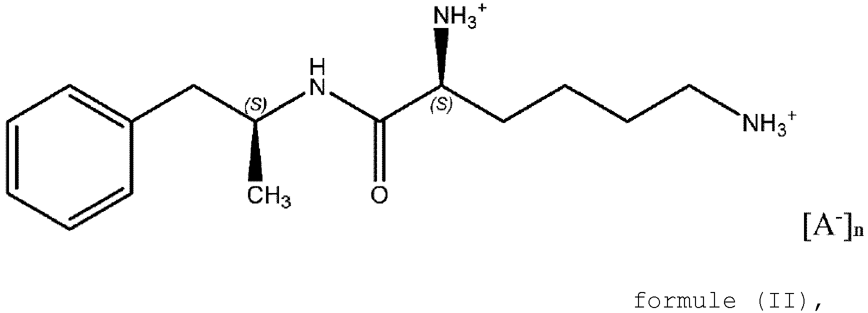 dans laquelle n est compris dans la plage allant de 1,9 à 2,1 et A est choisi parmi le groupe constitué d'acide adipique, d'acide *p*-toluène sulfonique, d'acide fumarique et d'acide oxalique, et dans laquelle le sel cristallin est choisi parmi le groupe constitué de
• diadipate *de* L-lysine-*d*-amphétamine (forme I), **caractérisé en ce qu'**il a un diffractogramme de poudre des rayons X comprenant des réflexions aux angles 2-Theta (4,7 ± 0,2)°, (11,1 ± 0,2)° et (18,9 ± 0,2)°, lorsqu'ils sont mesurés à une température comprise dans la plage allant de 20 à 30 °C et à une humidité relative comprise dans la plage allant de 0 à 65 % avec un rayonnement Cu-Kalpha_{1,2} ayant une longueur d'onde de 0,15419 nm ;
• diadipate de *L*-lysine-*d*-amphétamine (forme II), **caractérisé en ce qu'**il a un diffractogramme de poudre des rayons X comprenant des réflexions aux angles 2-Theta (4,7 ± 0,2)°, (7,1 ± 0,2)° et (21,1 ± 0,2)°, lorsqu'ils sont mesurés à une température comprise dans la plage allant de 20 à 30 °C et à une humidité relative comprise dans la plage allant de 0 à 65 % avec un rayonnement Cu-Kalpha_{1,2} ayant une longueur d'onde de 0,15419 nm ;
• ditosylate de *L*-lysine-*d*-amphétamine (forme I), **caractérisé en ce qu'**il a un diffractogramme de poudre des rayons X comprenant des réflexions aux angles 2-Theta (6,0 ± 0,2)°, (7,4 ± 0,2)° et (8,1 ± 0,2)°, lorsqu'ils sont mesurés à une température comprise dans la plage allant de 20 à 30 °C et à une humidité relative comprise dans la plage allant de 0 à 65% avec un rayonnement Cu-Kalpha_{1,2} ayant une longueur d'onde de 0,15419 nm ;
• ditosylate de *L*-lysine-*d*-amphétamine (forme II), **caractérisé en ce qu'**il a un diffractogramme de poudre des rayons X comprenant des réflexions aux angles 2-Theta (5,0 ± 0,2)°, (5,7 ± 0,2)° et (7,2 ± 0,2)°, lorsqu'ils sont mesurés à une température comprise dans la plage allant de 20 à 30 °C et à une humidité relative comprise dans la plage allant de 0 à 65% avec un rayonnement Cu-Kalpha_{1,2} ayant une longueur d'onde de 0,15419 nm ;
• difumarate de *L*-lysine-*d*-amphétamine, **caractérisé en ce qu'**il a un diffractogramme de poudre des rayons X comprenant des réflexions aux angles 2-Theta (4,3 ± 0,2)°, (6,6 ± 0,2)° et (23,8 ± 0,2)°, lorsqu'ils sont mesurés à une température comprise dans la plage allant de 20 à 30 °C et à une humidité relative comprise dans la plage allant de 0 à 65 % avec un rayonnement Cu-Kalpha_{1,2} ayant une longueur d'onde de 0,15419 nm ; et
• *L*-lysine-*d*-amphétamine dioxalate, **caractérisé en ce qu'**il a un diffractogramme de poudre des rayons X comprenant des réflexions aux angles 2-Theta (9,2 ± 0,2)°, (9,4 ± 0,2)° et (12,3 ± 0,2)°, lorsqu'ils sont mesurés à une température comprise dans la plage allant de 20 à 30 °C et à une humidité relative comprise dans la plage allant de 0 à 65% avec un rayonnement Cu-Kalpha_{1,2} ayant une longueur d'onde de 0,15419 nm.

2. Utilisation du sel cristallin tel que défini dans la revendication 1 pour la préparation d'une composition pharmaceutique.

3. Composition pharmaceutique comprenant le sel cristallin tel que défini dans la revendication 1 et au moins un excipient pharmaceutiquement acceptable.

4. Composition pharmaceutique selon la revendication 3, dans laquelle l'au moins un excipient pharmaceutiquement acceptable est choisi parmi le groupe constitué de cellulose microcristalline, de croscarmellose sodique et de stéarate de magnésium.

5. Composition pharmaceutique selon la revendication 3 ou 4, dans laquelle la composition pharmaceutique est une forme galénique orale solide.

6. Composition pharmaceutique selon la revendication 5, dans laquelle la forme galénique orale solide est une gélule.

7. Composition pharmaceutique selon la revendication 6, dans laquelle la gélule est une gélule en gélatine dure.

8. Sel cristallin tel que défini dans la revendication 1 ou la composition pharmaceutique selon l'une quelconque des revendications 3 à 7 pour utilisation comme médicament.

9. Le sel cristallin tel que défini dans la revendication 1 ou la composition pharmaceutique selon l'une quelconque des revendications 3 à 7 pour utilisation dans le traitement des symptômes du Trouble du Déficit de l'Attention avec Hyperactivité (TDAH) et d'hyperphagie boulimique (BED).
